# EUROPEAN PATENT SPECIFICATION

(11) **EP 3 429 521 B1**
(45) Date of publication and mention of the grant of the patent: **29.12.2021**
(21) Application number: 17711522.7
(22) Date of filing: 06.03.2017
(51) Int. Cl.: A61F 13/00, A61F 13/02, A61F 15/00

(54) **DRESSING AUGMENTATION TO PROMOTE EPITHELIALIZATION**
VERBANDSVERSTÄRKUNG ZUR FÖRDERUNG DER EPITHELIALISIERUNG
AUGMENTATION DE PANSEMENT POUR PROMOUVOIR L'ÉPITHÉLISATION

(30) Priority: 18.03.2016 US 201662310443 P
(43) Date of publication of application: 23.01.2019
(73) Proprietor: 3M Innovative Properties Company, St. Paul, MN 55133-3427 (US)
(72) Inventor: LUCKEMEYER, James, A., San Antonio, TX 78250 (US); COURAGE, James, San Antonio, TX 78232 (US); ROBINSON, Timothy, Mark, Shillingstone DT11 0TG (GB); LOCKE, Christopher, Brian, Bournemouth BH9 3SD (GB)
(74) Representative: Simmons & Simmons
(86) International application number: PCT/US2017/020875
(87) International publication number: WO 2017/160517

(56) References cited:
- EP-A2- 2 433 596
- WO-A1-2009/049232
- US-A1- 2005 096 574
- US-A1- 2014 188 058
- US-B1- 6 299 018

## Description

### TECHNICAL FIELD

The invention set forth in the appended claims relates generally to tissue treatment systems and more particularly, but without limitation, to apparatuses for promoting epithelialization at the perimeter of wounds during negative-pressure therapy.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times. WO 2009/049232 A1 concerns a surgical tissue therapy device that includes a sealant layer and a collection chamber.

EP 2 433 596 A2 concerns a dressing for covering a wound of a patient, for example in the case of a penetrating chest wound to assist in the patient's breathing, comprising a sheet member adapted to be adhered to the patient's skin in use to provide an airtight seal around the wound.

While the clinical benefits of negative-pressure therapy are widely known, improvements to negative-pressure systems, components, and processes may benefit healthcare providers and patients.

### BRIEF SUMMARY

There is provided a system for treating a tissue site in accordance with each of claims 1 and 2. Optional features are set out in the dependent claims.

Objectives, advantages, and a preferred mode of making and using the claimed subject matter may be understood best by reference to the accompanying drawings in conjunction with the following detailed description of illustrative embodiments useful for understanding the invention.

### BRIEF DESCRIPTION OF THE DRAWINGS

Figure 1 is a functional block diagram of an example embodiment of a therapy system that can deliver negative pressure to a tissue site in accordance with this specification;
Figure 2 is an assembly view illustrating additional details that may be associated with some example embodiments of a wound dressing in the therapy system of Figure 1;
Figure 3 is a schematic diagram of an example embodiment of a device that can be used to apply a tape to a tissue site; and
Figure 4 is a schematic diagram of an alternative example embodiment of a device that can be used to apply a tape to a tissue site.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

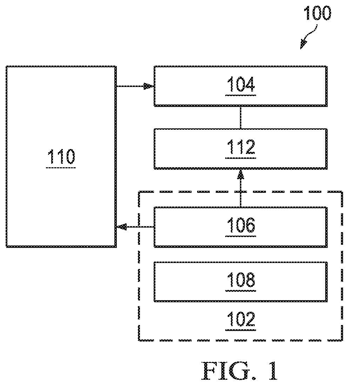

Figure 1 is a simplified functional block diagram of an example embodiment of a therapy system 100 that can provide negative-pressure therapy in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply, which may include or be configured to be coupled to a distribution component, such as a dressing. In general, a distribution component may refer to any complementary or ancillary component configured to be fluidly coupled to a negative-pressure supply in a fluid path between a negative-pressure supply and a tissue site. A distribution component is preferably detachable, and may be disposable, reusable, or recyclable. For example, a dressing 102 may be fluidly coupled to a negative-pressure source 104, as illustrated in Figure 1. The dressing 102 may include a cover 106 and a tissue interface 108. A regulator or a controller, such as a controller 110, may also be coupled to the negative-pressure source 104.

In some embodiments, a dressing interface may facilitate coupling the negative-pressure source 104 to the dressing 102. For example, such a dressing interface may be a T.R.A.C.^{®} Pad or Sensa T.R.A.C.^{®} Pad available from Kinetic Concepts, Inc. of San Antonio, Texas. The therapy system 100 may optionally include a fluid container, such as a container 112, coupled to the dressing 102 and to the negative-pressure source 104.

Components may be fluidly coupled to each other to provide a path for transferring fluids (i.e., liquid and/or gas) between the components. For example, components may be fluidly coupled through a fluid conductor, such as a tube. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112 in some embodiments.

In general, components of the therapy system 100 may be coupled directly or indirectly. For example, the negative-pressure source 104 may be directly coupled to the controller 110, and may be indirectly coupled to the dressing 102 through the container 112.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, in some embodiments, the negative-pressure source 104 may be combined with the controller 110 and other components into a therapy unit. A negative-pressure supply may also have one or more supply ports configured to facilitate coupling and de-coupling the negative-pressure supply to one or more distribution components.

The tissue interface 108 can be generally adapted to contact a tissue site. The tissue interface 108 may be partially or fully in contact with the tissue site. If the tissue site is a wound, for example, the tissue interface 108 may partially or completely fill the wound, or may be placed over the wound. The tissue interface 108 may take many forms, and may have many sizes, shapes, or thicknesses depending on a variety of factors, such as the type of treatment being implemented or the nature and size of a tissue site. For example, the size and shape of the tissue interface 108 may be adapted to the contours of deep and irregular shaped tissue sites. Moreover, any or all of the surfaces of the tissue interface 108 may have projections or an uneven, course, or jagged profile that can induce strains and stresses on a tissue site, which can promote granulation at the tissue site.

In some embodiments, the tissue interface 108 may be a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, the pathways of a manifold may be interconnected to improve distribution or collection of fluids across a tissue site. In some illustrative embodiments, a manifold may be a porous foam material having interconnected cells or pores. For example, cellular foam, open-cell foam, reticulated foam, porous tissue collections, and other porous material such as gauze or felted mat generally include pores, edges, and/or walls adapted to form interconnected fluid channels. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

The average pore size of a foam may vary according to needs of a prescribed therapy. For example, in some embodiments, the tissue interface 108 may be a foam having pore sizes in a range of 400-600 microns. The tensile strength of the tissue interface 108 may also vary according to needs of a prescribed therapy. For example, the tensile strength of a foam may be increased for instillation of topical treatment solutions. In one non-limiting example, the tissue interface 108 may be an open-cell, reticulated polyurethane foam such as GranuFoam^{®} dressing or VeraFlo^{®} foam, both available from Kinetic Concepts, Inc. of San Antonio, Texas.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of a hydrophilic foam is a polyvinyl alcohol, open-cell foam such as V.A.C. WhiteFoam^{®} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

The tissue interface 108 may further promote granulation at a tissue site when pressure within the sealed therapeutic environment is reduced. For example, the tissue interface 108 may have at least one granulating surface that can induce microstrains and stresses at a tissue site if negative pressure is applied through the tissue interface 108. A granulating surface generally provides an uneven, coarse, or jagged profile.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include without limitation polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 106 may provide a bacterial barrier and protection from physical trauma. The cover 106 may also be constructed from a material that can reduce evaporative losses and provide a fluid seal between two components or two environments, such as between a therapeutic environment and a local external environment. The cover 106 may be, for example, an elastomeric film or membrane that can provide a seal adequate to maintain a negative pressure at a tissue site for a given negative-pressure source. The cover 106 may have a high moisture-vapor transmission rate (MVTR) in some applications. For example, the MVTR may be at least 300 g/m^2 per twenty-four hours in some embodiments. In some example embodiments, the cover 106 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained.

An attachment device may be used to attach the cover 106 to an attachment surface, such as undamaged epidermis, a gasket, or another cover. The attachment device may take many forms. For example, an attachment device may be a medically-acceptable, pressure-sensitive adhesive that extends about a periphery, a portion, or an entire sealing member. In some embodiments, for example, some or all of the cover 106 may be coated with an acrylic adhesive having a coating weight between 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

A controller, such as the controller 110, may be a microprocessor or computer programmed to operate one or more components of the therapy system 100, such as the negative-pressure source 104. In some embodiments, for example, the controller 110 may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the tissue interface 108, for example. The controller 110 is also preferably configured to receive one or more input signals, such as a feedback signal, and programmed to modify one or more operating parameters based on the input signals. For example, the controller 110 may receive signals from one or more sensors, such as a pressure sensor or electric sensor, that are operable to detect or measure one or more operating parameters of the therapy system 100.

The container 112 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

In operation, the tissue interface 108 may be placed within, over, on, or otherwise proximate to a tissue site. The cover 106 may be placed over the tissue interface 108 and sealed to an attachment surface near the tissue site. For example, the cover 106 may be sealed to undamaged epidermis peripheral to a tissue site. Thus, the dressing 102 can provide a sealed therapeutic environment proximate to a tissue site, substantially isolated from the external environment, and the negative-pressure source 104 can reduce the pressure in the sealed therapeutic environment. Negative pressure applied across the tissue site through the tissue interface 108 in the sealed therapeutic environment can induce macrostrain and microstrain in the tissue site, as well as remove exudates and other fluids from the tissue site, which can be collected in container 112.

Wound therapy systems, such as the therapy system 100, can be used to promote granulation of a tissue site, and treatment protocols often involve following negative-pressure therapy with other dressings to complete epithelial closure of the tissue site. During negative-pressure therapy, which may be applied with negative-pressure systems, such as therapy system 100, epithelialization may actually be delayed due to several factors. First, caregivers often have a tendency to over pack wounds with foam, which can lead to irritation of the wound edges. Second, wound edges may experience drape shear due to vacuum forces, in addition to patient movement. Furthermore, damage to epithelial growth often occurs during drape and foam removal, such as during routine wound dressing changes. With deep wounds, the primary pain sensations may be around the wound edges. Thus, there is a need for a wound dressing enhancement that protects the edges of a wound site from disruption, and thereby accelerates the naturally-occurring epithelialization and healing processes of a wound site, while also minimizing irritation and discomfort for patients. As disclosed herein, the therapy system 100 may address these needs and others. For example, the therapy system 100 may provide a smooth, sheer-less surface along the perimeter of a wound, without compromising granulation in the middle. By actively supporting epithelialization earlier in the wound healing process, the overall time to closure of the wound should be reduced.

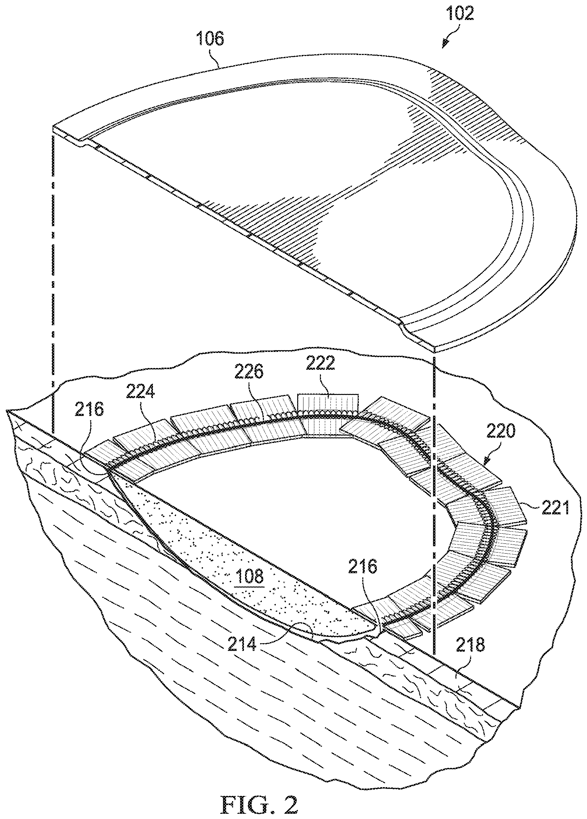

Figure 2 is a schematic view illustrating additional details that may be associated with some example embodiments of the dressing 102. In the example embodiment of Figure 2, the dressing 102 generally includes the cover 106, the tissue interface 108, as well as an epithelialization interface in the form of a tape 220. The tape 220 may be applied around the circumference of a wound site 214, along the wound edge 216. Some embodiments of the tape 220 may comprise or be substantially formed from a biocompatible material, which may have a smooth, low-friction surface. For example, the tape 220 may be formed from a hydrogel having a low viscosity, which may allow for any shear forces applied to the tape 220 to be dispersed and/or absorbed by the tape 220, and largely not transmitted to the wound site 214.

Additionally, the tape 220 may offer semi-adherent or substantially non-adherent surfaces, which may allow the tape 220 to remain in place while the other components of the dressing 102, such as the cover 106, are changed. For example, the tape 220 may include a tissue-facing surface and an upper surface. The upper surface of the tape 220 may be semi-adherent in order to support a high seal with the cover 106, however the bond between the upper surface of the tape 220 and the cover 106 may be insufficient to cause the tape 220 to be removed with the cover 106 and/or the tissue interface 108, when these components of the dressing 102 are changed. The tape 220 may also be capable of maintaining a moist environment. For example, in some embodiments, the tape 220 may be adapted to absorb excess moisture around the edges of the wound site 214, as well as donate water back to the wound site 214 in more dry conditions. Therefore, in some preferred embodiments, the tape 220 may include a material that can absorb at least its own weight in water.

In the example embodiment of Figure 2, the tape 220 may be in the form of tape strips, such as segments 221, and may be formed from a hydrogel 222 and reinforced with an optional internal textile scrim 224. In some embodiments, the hydrogel 222 may be a hydrophilic material with a viscosity range of 15 shore 000 to 40 shore 000. The hydrogel 222 may have a low-friction surface and may be adapted to maintain a moist environment at a wound site, such as wound site 214, and thus may not hinder the migration of cells. In some embodiments, the hydrogel 222 may include materials such as the LUDLOW product, available from Covidien, or the AQUACLEAR product, available from HARTMANN USA, Inc. In other embodiments, the tape 220 may be formed from alternatives to a hydrogel, and may include one or more of the following materials: cyanoacrylate sealant, fibrin sealant, silicone sealant, collagen sealant, hydrocolloid sealant, albumin sealant, hot-melt-type surgical paste, polymer gels such as polyurethanes, polyolefins, acrylics, and cellulosic (carboxymethyl cellulose), and alginates. The materials typically have a smooth, low-friction surface, are capable of maintaining a moist environment, and are biocompatible. Example embodiments of the tape 220 may include Adaptic^{®} product from Systagenix Wound Management, ALGIDERM product, available from C. R. Bard, Inc., and/or AQUACEL product, available from ConvaTec, Inc.

The textile scrim 224 may prevent excessive stretching of the tape 220 and may provide support for the edge of the tape 220, as it can overhang the wound edge 216 into the wound site 214 in some applications. The textile scrim 224 may take various forms, but in some preferred embodiments may be a mesh or net, and may include fringes that span throughout the tape 220. In some embodiments, the textile scrim 224 may be an elastic woven or non-woven material. In some embodiments, the textile scrim 224 may comprise or consist essentially of one or more polyurethane elastomers, for example, elastane or spandex materials. The textile scrim may be of various dimensions and weights, and in some embodiments, may be about 25 g/m². A tape centerline marker 226 may also be incorporated into the tape 220, which may assist a user with following the perimeter of a wound when applying the tape 220 to the wound edge 216. In some embodiments, the tape centerline marker 226 may be a colored thread.

The tape 220 may be of any suitable width or thickness, but in some preferred embodiments, may be in the range of 20 mm to 35 mm wide, with a thickness of 1 mm to 2 mm. In some embodiments, the width dimension may be influenced by usability studies of the optimal offset of the tape 220 along the inside edge of a wound, such as wound site 214, which may encourage decreased sizing of a tissue interface (to avoid over-packing the wound bed), such as tissue interface 108, as wound healing progresses. In other embodiments, the tape 220 may include the feature of a dimensional bias to the tape centerline marker 226 away from a center line of the tape 220. Such a feature may help guide and train users. For example, users may be instructed to put the wide edge of the tape 220 towards the inside of the wound edge 216 for large wounds, or the narrow edge of the tape 220 to the inside of the wound edge 216 for small wounds. Further, in some embodiments, the tape 220 may be offered in different color choices, which may allow users to personalize the product, as well as may be used to color-code different sizes or varieties of the tape 220.

The tape 220 may provide various structural benefits to the perimeter of a wound, such as along the wound edge 216. For example, the tape 220 may prevent tension from the cover 106 or other component of the dressing 102 from being transferred to the wound edge 216, thereby preventing shear forces from contacting the wound edge 216. Furthermore, in some embodiments, the tape 220 may be formed with adequate strength to resist collapse under a compressive force transmitted by the cover 106 when negative pressure is applied to the dressing 102.

Additionally, the physical structure and properties of the tape 220 may offer significant benefits for application to non-linear wounds, such as circular or irregularly-shaped wounds. Whereas typically, attempting to apply a linear-type tape or material around bends or corners would result in an uneven surface with ridges, possibly leading to discontinuous contact with a tissue site, and consequently, leaks, the properties of the tape 220 may allow it to conform to a variety of differently-shaped wounds. For example, as depicted in Figure 2, the tape 220 may be in the form of a segmented structure, which may improve the ability of the tape 220 to flex, bend, or otherwise conform to the perimeter of a wound. In some embodiments, the tape 220 may include portions, such as segments 221, that are independently positionable, for closely tracking an irregularly-shaped border of a wound. In some instances, the tape 220 may include perforations between the segments 221, which may allow the segments 221 to be individually shaped to the contours of a wound. Additionally, in some embodiments, the segments 221 may be individually removable. To provide even greater flexibility, the tape 220, may be offered with segments 221 having a variety of sizes. For example, in some embodiments, the tape 220 may have segments 221 that are of a shorter length, therefore allowing for even a more precise fit around edges of small and/or particularly irregularly-shaped wounds. In some additional embodiments of the tape 220, some segments 221 may be of a greater length, while some may be of a smaller length, and may be in an alternating arrangement.

Another benefit of the segmented structure of the tape 220 is that, as compared to a more traditional configuration of adhesive tape being applied to the external surface of a wound drape for securing the drape to a patient, the tape 220 may be much more user-friendly. For example, while portions of a traditional adhesive tape may have a high tendency to stick to each other, and consequently leading to the tape being ruined and discarded, the segments 221 of the tape 220 can be easily positioned around, as well as in contact with, each other without becoming permanently stuck to each other. As a result, the tape 220 may be much more forgiving to a user who may be inexperienced or who may need to make adjustments during the application of the tape 220, in order to achieve the best application around a given wound site.

Furthermore, in some embodiments, the textile scrim 224 of the tape 220 may be at least partially elastic or stretchable in nature, which may further assist a user, such as a clinician, with tracing irregular borders of a wound, such as the wound edge 216. Thus, at least in some embodiments, due to the combination of potentially elastic properties and the small width of the textile scrim 224 relative to the overall tape 220, the tape 220 may be particularly effective at making a close contact around sharp corners of a wound site, despite originating as a linear material. As a result, an improved seal may be formed between the tape 220 and the cover 106, when applied, which may help reduce or prevent fluid leaks.

Still referring to Figure 2, the tissue interface 108 may be placed in contact with the wound site 214, inside of the boundary formed by the tape 220 that is applied to the wound edge 216. The cover 106 may be positioned over the tape 220 and tissue interface 108, and a portion of the epidermis 218. As discussed in further detail above, the cover 106 may be attached to the epidermis 218 surrounding the wound site 214, using a variety of conventional attachment mechanisms.

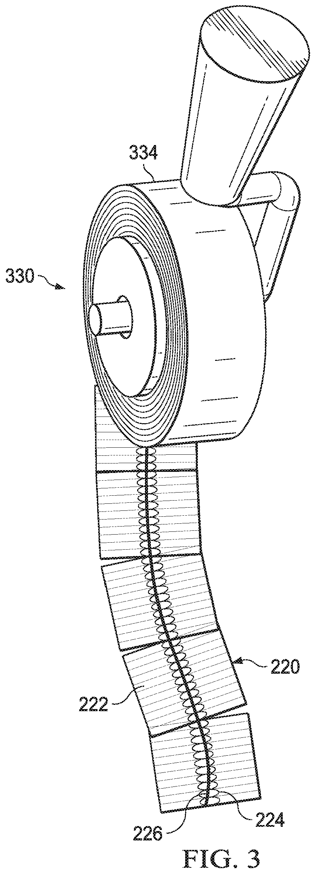

Figure 3 is a perspective view of an example embodiment of an applicator that may be used with the tape 220. The applicator may offer various benefits, for example, such as allowing the tape 220 to be applied more quickly. In the example of Figure 3, the applicator is a tape roller 330. In operation, the tape 220 may be applied around a perimeter of a wound by rolling the tape roller 330 around a wound edge. The tape centerline marker 226 may assist with tracking the wound edge 216. The applied tape 220 may then provide a visual cue for proper sizing of a tissue interface, such as the tissue interface 108, to fit inside of the tape edge, which can discourage over-packing the wound site 214 and avoid contact of the tissue interface 108 with the wound edge 216. In some embodiments the tape roller 330 may include components that are injection-molded polymers, such as PC (polycarbonate), ABS (acrylonitrile-butadiene-styrene), or PC-ABS blends. PC-ABS blends may include the C1200HF, C2800, or C2950 products, as supplied by SABIC, or alternatively, may include the Emerge 7570 or Pulse 2000EZ products, as supplied by TRINSEO. In other embodiments, the tape roller 330 may include a construction including combinations of rigid plastics and thermoplastic elastomers. The applicator may be a mini tape roller, such as the tape roller 330, and may be a disposable medical product, which may be furnished sterile. Further, in some embodiments, the entire applicator assembly, along with any remaining tape, may be disposed after a single patient use. In some circumstances, remaining tape may be used to assist with sealing drape leaks during subsequent drape overlay. To aid such use at a later time, the spool, such as tape spool 334, could be designed to easily pop-off from the tape roller, such as tape roller 330.

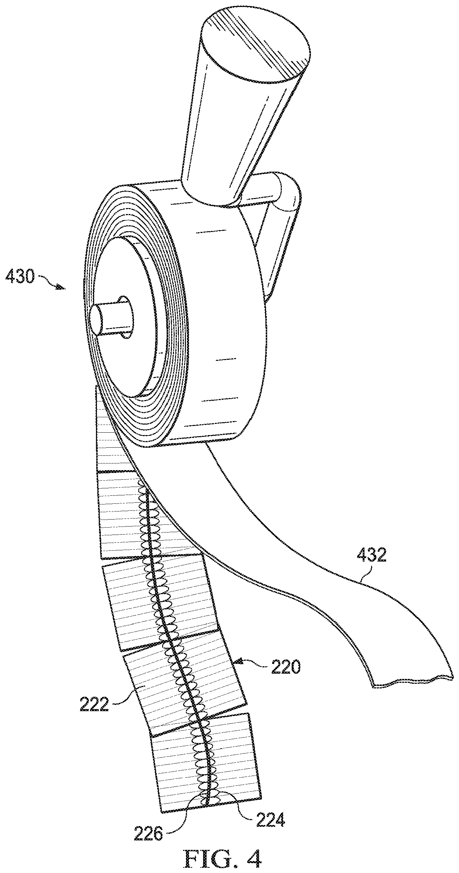

Figure 4 is a perspective view of an alternative embodiment of a tape roller 430 that may be used to apply tape, such as tape 220, to a tissue site. The tape roller 430 may be used with the tape 220, as well as an additional separating layer 432 for ease of applying a single layer of tape 220 at a time from the tape roller 430. For example, in some embodiments, the separating layer 432 may be a thin silicone layer. When applied to a wound edge, the separating layer 432 may also provide a silicone surface for reducing attachment of the cover 106. Thus, the tape 220 may remain in place during a dressing change, which may further reduce trauma to new epithelial cell growth. In some embodiments, the upper sealing face of the separating layer 432 may be made from a silicone gel, elastomer, or other material unlikely to bond well to an acrylic adhesive. In some embodiments, the separating layer 432 may have an upper layer of self-lubricating silicone, which may excrete small volumes of liquid silicone over the wear duration. Such materials are known in the art and may be obtained from suppliers such as WACKER, MOMENTIVE, and DOW. In some embodiments, the upper sealing face of the separating layer 432 may include scrim material or support fabric, which may allow the separating layer 432 to mechanically link with adhesive on the surface of a cover, while also allowing easy separation of the separating layer 432 from the cover, such as cover 106.

The separating layer 432 may also provide a seal for the dressing 102, as there may be some degree of bond between the cover 106 and the separating layer 432. However, this bond may be insufficient to cause the separating layer 432, or the tape 220 as a whole, to be removed with the cover 106 when portions of the dressing are changed. In some embodiments, the separating layer 432 may be left in-place for several dressing changes. For example, the tape 220 may be changed every second or third dressing change. The tape 220 may also have the inherent feature of providing guidance during periodic reapplications if the tape 220 is reapplied with a smaller perimeter around a wound site, thus enforcing the resizing of a wound interface, such as a foam, to shrink as the wound healing progresses.

In addition to the tape embodiments already discussed, the tape concept can be extended to include various additional features and therapies. For example, in some embodiments, human dermis tissue may also be included as part of the tape, as hydrogels have been shown in the art to be a suitable media for allowing epithelial cell growth and extracellular matrix formation. In such embodiments, tape strips may be preferred to a roll of tape, as the geometry of the tape strips may be more suitable for inclusion of human dermal cells. Dermal-spiked tape strips may be placed around a wound perimeter, or selectively in areas in or around a wound site that are ready for epithelialization. In some embodiments, additional tape strips, or tape applied from a tape roller, may be used to trace areas ready for epithelialization, particularly in cases of larger overall wound sites.

In other alternative embodiments, the tape may incorporate features to allow electrical stimulation only in the tape area. In such embodiments, the electrical stimulation may be provided by galvanic cells or an externally-applied source. For example, in some embodiments, a conductive element, such as a carbon-impregnated fiber, conductive polymer thread, or thin metallic wire may be placed near each edge of the tape such that an electrical current source can be applied to provide electrical stimulation across the tape, in the desired direction of epithelialization along the wound edge. In other embodiments, the conductor materials could be chosen to create a galvanic cell across the tape width, taking advantage of the salts and ions in wound fluid. In some embodiments, for electrical stimulation, the electrical source could be an external battery for continuous stimulation, or in other alternative embodiments, the electrical source could be a battery with an electronic circuit to generate a preferred waveform and control timing for repeated stimulation sessions. In such embodiments, the electronic circuit may be a small electronic circuit that may be placed at the wound site. Still in other alternative embodiments, electrical contacts may be included for attachment of an electronic-stimulation therapy device for periodic treatment sessions performed by a caregiver.

In some alternative embodiments, the tape construct may also incorporate a silk mat designed to slowly release a therapeutic substance, such as growth factors, for example, epidermal growth factor (EGF). In other alternative embodiments, nanostructures may similarly be incorporated into the tape construct, which may adapted to release drugs and other active agents. In still other alternative embodiments, antimicrobial or anti-biofilm features could be added to the tape construct. For example, there are hydrogels known in the art designed to break down biofilms by damaging microbe membranes through electrostatic interaction of positively-charged gel and negatively-charged bacterial or fungal cells. In other embodiments, electro-spun fiber structures designed to attract and capture infectious bacteria may be incorporated in the tape construct.

Further, in some alternative embodiments, the tape may employ a color-changing feature to alert caregivers of an infection present at the wound site. For example, the tape may include a temperature-sensitive material that may change colors in response to temperature. In other embodiments, the tape may include electro-spun fiber structures designed to attract and capture infectious bacteria, which may change color with pH change. Such features may be particularly helpful for untrained users or caregivers in the home healthcare market. In some additional embodiments, the hydrogel for tape materials could also be designed to provide mechanical stimulation of tissue along the perimeter of the wound site. For example, hydrogels can be designed to include ionic conduction properties, and may be capable of generating large strains.

The systems, apparatuses, and methods described herein may provide significant advantages, many of which have already been discussed. Generally, the tape embodiments may promote an acceleration of wound closure through a variety of features and mechanisms. For example, when applied in the context of negative-pressure therapy, the tape may encourage epithelialization while the negative-pressure wound therapy promotes granulation, rather than having these processes separated into two discrete wound closure therapy steps.

The tape may also provide significant benefits to areas surrounding the wound that may typically be in contact with wound dressing covers. For example, the tape may help manage adherence of a wound cover, such as a drape, along the perimeter of the wound. The tape may both improve drape sealing to the wound perimeter to improve the application of negative-pressure wound therapy, as well as help minimize or avoid cellular trauma during dressing changes, particularly in the context of negative-pressure wound therapy. Additionally, the tape, when properly positioned, has the additional benefit of preventing drape shear along the wound epithelial edges to avoid damaging delicate cells on the surface of the healing wound edges. Furthermore, the tape may be constructed such that drape shear forces, which may result from multiple factors, do not transfer to the skin surface. For example, such forces may result from stretching and wrinkling due to the application of negative pressure to the wound dressing, or may be due to externally-applied shear forces during patient movement. The tape may also minimize or eliminate epithelial cell damage along the wound edge during drape and tissue interface removal and changes. The tape may also improve sealing, which may be beneficial for negative-pressure therapy, as well as optimize moisture management along the wound edge, which may help provide a preferred cellular environment for epithelialization.

Additionally, the tape embodiments disclosed herein may have benefits for users or caregivers. For example, some embodiments of the tape may be quickly applied with one hand, which may be less messy for a user than the use of a lotion product. The tape may also be beneficial for use as a sealing material in conjunction with negative-pressure wound therapy placements with wounds that are in body contours or other challenging locations which may be prone to leaks. In some embodiments, the tape may also provide training benefits for caregivers, as the tape may provide guidance for appropriate sizing of wound dressing components, such as a tissue interface or foam. For example, the tape may guide users to gradually reduce the size of a foam with each dressing change, to help counteract the clinically-improper tendency to overfill wounds with foam or other form of tissue interface.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 102, the container 112, or both may be eliminated or separated from other components for manufacture or sale. In other example configurations, the controller 110 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

## Claims

1. A system (100) for treating a tissue site, comprising:
a tape (220) comprising a flexible material for placing in contact with a perimeter of the tissue site, wherein the flexible material of the tape comprises a hydrogel (222) including a non-adherent upper surface and a tissue-facing surface in the form of segments (221) formed from the hydrogel (222) and reinforced with an internal textile scrim (224);
a tissue interface (108) comprising a granulating surface for placing in contact with the tissue site, inside of a boundary formed by the tape (220);
a cover (106) for positioning over the tape (220) and tissue interface (108) and a portion of an epidermis surrounding the tissue site, wherein the cover (106) has an adherent material comprising a medically-acceptable, pressure-sensitive adhesive that extends about a periphery of the cover (106) for coupling to the epidermis surrounding the tissue site and operable to form a fluid seal, and wherein the cover (106) is non-adherent to the non-adherent upper surface of the tape (220); and
a negative-pressure subsystem (104) for providing negative pressure to the tissue interface.

2. A system (100) for treating a tissue site, comprising:
a tape (220) comprising a flexible material for placing in contact with a perimeter of the tissue site, wherein the flexible material of the tape comprises a hydrogel (222) including a non-adherent upper surface and a tissue-facing surface in the form of segments (221) formed from the hydrogel (222) and reinforced with an internal textile scrim (224);
a tissue interface (108) comprising a granulating surface for placing in contact with the tissue site, inside of a boundary formed by the tape (220);
a cover (106) for positioning over the tape (220) and tissue interface (108) and a portion of an epidermis surrounding the tissue site, wherein the cover (106) has an adherent material for coupling to the epidermis surrounding the tissue site and operable to form a fluid seal; and
a negative-pressure subsystem (104) for providing negative pressure to the tissue interface
wherein the tape further comprises a separating layer (432) comprising a silicone layer applied to a surface of the flexible material for reducing attachment of the cover (106) such that the cover (106) is non-adherent to the non-adherent upper surface of the tape (220).

3. The system of any preceding claim, wherein the cover (106) is transparent.

## Patentansprüche

1. System (100) zur Behandlung einer Gewebestelle, umfassend:
ein Band (220), das ein flexibles Material zum Platzieren in Kontakt mit einem Umfang der Gewebestelle umfasst, wobei das flexible Material des Bandes ein Hydrogel (222) umfasst, das eine nicht haftende obere Oberfläche und eine gewebezugewandte Oberfläche in Form von Segmenten (221) einschließt, die aus dem Hydrogel (222) gebildet und mit einem inneren Textilmull (224) verstärkt sind;
eine Gewebeschnittstelle (108) umfassend eine granulierende Oberfläche zum Platzieren in Kontakt mit der Gewebestelle innerhalb einer durch das Band (220) gebildeten Grenze;
eine Abdeckung (106) zum Positionieren über dem Band (220) und der Gewebeschnittstelle (108) und einem Abschnitt einer die Gewebestelle umgebenden Epidermis, wobei die Abdeckung (106) ein anhaftendes Material aufweist, das einen medizinisch verträglichen, druckempfindlichen Klebstoff umfasst, der sich um einen Umfang der Abdeckung (106) zum Koppeln mit der Epidermis, welche die Gewebestelle umgibt, erstreckt und betreibbar ist, um eine Fluiddichtung zu bilden, und wobei die Abdeckung (106) nicht an der nicht haftenden oberen Oberfläche des Bandes (220) haftet; und
ein Unterdruck-Subsystem (104) zum Bereitstellen von Unterdruck an die Gewebeschnittstelle.

2. System (100) zur Behandlung einer Gewebestelle, umfassend:
ein Band (220), das ein flexibles Material zum Platzieren in Kontakt mit einem Umfang der Gewebestelle umfasst, wobei das flexible Material des Bandes ein Hydrogel (222) umfasst, das eine nicht haftende obere Oberfläche und eine gewebezugewandte Oberfläche in Form von Segmenten (221) einschließt, die aus dem Hydrogel (222) gebildet und mit einem inneren Textilmull (224) verstärkt sind;
eine Gewebeschnittstelle (108) umfassend eine granulierende Oberfläche zum Platzieren in Kontakt mit der Gewebestelle innerhalb einer durch das Band (220) gebildeten Grenze;
eine Abdeckung (106) zum Positionieren über dem Band (220) und der Gewebeschnittstelle (108) und einem Abschnitt einer die Gewebestelle umgebenden Epidermis, wobei die Abdeckung (106) ein anhaftendes Material zum Koppeln an die die Gewebestelle umgebende Epidermis aufweist und betreibbar ist, um eine Fluiddichtung zu bilden; und
ein Unterdruck-Subsystem (104) zum Bereitstellen von Unterdruck an die Gewebeschnittstelle
wobei das Band weiter eine Trennschicht (432) umfasst, die eine Silikonschicht umfasst, die auf eine Oberfläche des flexiblen Materials aufgebracht wird, um die Anbringung der Abdeckung (106) zu reduzieren, so dass die Abdeckung (106) nicht an der nicht haftenden oberen Oberfläche des Bandes (220) haftet.

3. System nach einem der vorstehenden Ansprüche, wobei die Abdeckung (106) transparent ist.

## Revendications

1. Système (100) de traitement d'un site tissulaire, comprenant :
une bande (220) comprenant un matériau flexible destinée à être placée en contact avec un périmètre du site tissulaire, dans lequel le matériau flexible de la bande comprend un hydrogel (222) incluant une surface supérieure non adhésive et une surface orientée vers le tissu sous forme de segments (221) formées à partir de l'hydrogel (222) et renforcées par une gaze textile interne (224) ;
une interface tissulaire (108) comprenant une surface de granulation destinée à être placée en contact avec le site tissulaire, à l'intérieur d'une limite formée par la bande (220) ;
une protection (106) destinée à être positionnée sur la bande (220) et l'interface tissulaire (108) et une portion d'un épiderme entourant le site tissulaire, dans lequel la protection (106) présente un matériau adhésif comprenant un adhésif autocollant médicalement acceptable qui s'étend sur une périphérie de la protection (106) destiné à se coupler à l'épiderme entourant le site tissulaire et pouvant fonctionner pour former un joint étanche aux fluides, et dans lequel la protection (106) n'adhère pas à la surface supérieure non adhésive de la bande (220) ; et
un sous-système à pression négative (104) destiné à fournir une pression négative à l'interface tissulaire.

2. Système (100) de traitement d'un site tissulaire, comprenant :
une bande (220) comprenant un matériau flexible destinée à être placée en contact avec un périmètre du site tissulaire, dans lequel le matériau flexible de la bande comprend un hydrogel (222) incluant une surface supérieure non adhésive et une surface orientée vers le tissu sous forme de segments (221) formées à partir de l'hydrogel (222) et renforcées par une gaze textile interne (224) ;
une interface tissulaire (108) comprenant une surface de granulation destinée à être placée en contact avec le site tissulaire, à l'intérieur d'une limite formée par la bande (220) ;
une protection (106) destinée à être positionnée sur la bande (220) et l'interface tissulaire (108) et une portion d'un épiderme entourant le site tissulaire, dans lequel la protection (106) présente un matériau adhésif destiné à se coupler à l'épiderme entourant le site tissulaire et pouvant fonctionner pour former un joint étanche aux fluides ; et
un sous-système à pression négative (104) destiné à fournir une pression négative à l'interface tissulaire
dans lequel la bande comprend en outre une couche de séparation (432) comprenant une couche de silicone appliquée sur une surface du matériau flexible pour réduire la fixation de la protection (106) de sorte que la protection (106) n'adhère pas à la surface supérieure non adhésive de la bande (220).

3. Système selon une quelconque revendication précédente, dans lequel la protection (106) est transparente.
